# EUROPÄISCHE PATENTSCHRIFT

(11) **EP 3 755 299 B1**
(45) Veröffentlichungstag und Bekanntmachung des Hinweises auf die Patenterteilung: **30.03.2022**
(21) Anmeldenummer: 19707715.9
(22) Anmeldetag: 14.02.2019
(51) Int. Cl.: A61J 1/10, A61J 1/20, B65B 3/00, B65B 7/00

(54) **VORRICHTUNG UND VERFAHREN ZUM BEFÜLLEN VON LÖSUNGSBEUTELN FÜR DIE DIALYSE**
APPARATUS AND METHOD FOR FILLING SOLUTION BAGS FOR DIALYSIS
DISPOSITIF ET PROCÉDÉ DE REMPLISSAGE DE POCHES DE SOLUTION POUR DIALYSE

(30) Priorität: 21.02.2018 DE 102018103863
(43) Veröffentlichungstag der Anmeldung: 30.12.2020
(73) Patentinhaber: Fresenius Medical Care Deutschland GmbH, 61352 Bad Homburg (DE)
(72) Erfinder: WABEL, Peter, 61191 Rosbach (DE); BREUNINGER, Marcus, 61352 Bad Homburg (DE); BERLICH, Robert, 66606 St. Wendel (DE); STAUDE, Birgit, 64319 Pfungstadt (DE); RAU, Matthias, 65195 Wiesbaden (DE); CERMAN, Zdenek, 65126 Idstein (DE)
(74) Vertreter: Herrmann, Uwe
(86) Internationale Anmeldenummer: PCT/EP2019/053661
(87) Internationale Veröffentlichungsnummer: WO 2019/162181

(56) Entgegenhaltungen:
- WO-A1-2017/127632
- US-A- 4 905 450
- US-A1- 2002 023 409
- US-A1- 2002 124 526

## Beschreibung

Die Erfindung betrifft eine Vorrichtung und Verfahren zum Befüllen von Lösungsbeuteln für die Dialyse.

Insbesondere im Bereich der Peritonealdialyse ist es üblich, dem Patienten Lösungsbeutel zur Verfügung zu stellen, die mit einer für den Patienten geeigneten Dialyselösung befüllt sind. Diese Lösungsbeutel schließt der Patient dann selbsttätig oder mit Unterstützung von medizinischem Fachpersonal an einen Einlaufschlauch an, um das Peritoneum mit der Lösung zu füllen.

Um die Beutel zu Befüllen, wird medizinische Flüssigkeit durch einen Befüllschlauch oder durch eine sonstige Befüllöffnung des Beutels eingeleitet und der Befüllschlauch bzw. die Öffnung sodann versiegelt. In der WO 2017/127632 A1 wird eine Vorrichtung zur Befüllung derartiger Beutel offenbart.

Aufgabe der Erfindung ist es, eine Vorrichtung zum Befüllen von Lösungsbeuteln für die Dialyse bereitzustellen, mit der eine sterile Befüllung und eine anschließende Versiegelung des Befüllschlauchs bzw. der Befüllöffnung auf einfache Weise erreicht werden kann.

Vor diesem Hintergrund betrifft die Erfindung eine Vorrichtung zum Befüllen eines Lösungsbeutels für die Dialyse mit einer medizinischen Flüssigkeit gemäß Anspruch 1. Die Vorrichtung umfasst vorzugsweise einen Befüllplatz mit der Nadel zum Einführen in eine Befüllleitung oder -öffnung des Beutels, wobei es sich bei der Nadel um eine doppelwandige Nadel mit einer vorzugsweise wärmeleitenden Außenwand und einer Innenwand handelt, wobei die Außenwand und die Innenwand thermisch voneinander isoliert sind, so dass zuverlässig unterschiedliche Temperaturen zwischen Außen- und Innenwand realisierbar sind.

Die Innenwand umgibt den Flüssigkeitskanal der Nadel. Aufgrund der vorzugsweise wärmeleitenden Eigenschaften der Außenwand kann diese besonders gut erhitzt werden, auch durch ein von der zu erhitzenden Stelle entfernt angeordnetes Heizelement.

Das Heizelement, das mit der Außenwand unmittelbar oder mittelbar in Verbindung steht ist so ausgeführt, dass es die Temperatur der Außenwand, insbesondere von deren Außenseite auf einen Wert oberhalb der Temperatur der Innenwand bringen kann.

Ein Erhitzen kann einerseits vor und nach jedem Befüllvorgang oder für mehrere Befüllvorgänge zur Desinfektion durchgeführt werden.

Ferner kann die Nadel auch erhitzt werden, vor und/oder während sie sich in der Befüllleitung oder -öffnung befindet. Dieses Erhitzen findet vorzugsweise auf eine Temperatur statt, bei der Keime bzw. Erreger abgetötet werden.

Vorzugsweise wird beim Erhitzen eine Temperatur gewählt, um an der Innenoberfläche der Befüllleitung oder -öffnung das Kunststoffmaterial des Lösungsbeutels aufzuschmelzen und eine einfache Druckversiegelung des Beutelmaterials nach Entfernen der Nadel zu ermöglichen. Durch die thermische Isolierung wird ein Aufheizen der Nadelaußenwand nicht durch die im Flüssigkeitskanal der Nadel befindliche Flüssigkeit behindert und umgekehrt die in den Lösungsbeutel einzuführende Flüssigkeit nicht unerwünscht erwärmt, was ggf. zu deren Schädigung führen würde.

In einer Ausführungsform Ist vorgesehen, dass die Außenwand der Nadel aus Metall besteht. Metall ist nicht nur thermisch sondern auch elektrisch leitend. Insbesondere ferromagnetische Metalle können geeignet sein. Es kann Induktiv erhitzt werden oder zum Erhitzen mit elektrischem Strom durchflossen werden. In einer Ausführungsform bestehen sowohl die Innenwand als auch die Außenwand aus Metall.

Grundsätzlich ist von der Erfindung auch der Einsatz von Kunststoff für die Außen- und/oder Innenwand der Nadel umfasst.

In einer Ausführungsform ist vorgesehen, dass es sich bei der thermischen Isolierung um eine Vakuumisolierung handelt. Mit einer Vakuumisolation kann eine besonders gute thermische Isolationswirkung erreicht werden. Alternativ kann zwischen den Wänden auch ein Isolationsgas bzw. Luft eingefüllt sein oder ein Isoliermaterial wie beispielsweise ein Keramikmaterial, Kunststoffmaterial, Fasermaterial oder Schaum angeordnet werden.

In einer Ausführungsform Ist vorgesehen, dass an der Außenoberfläche der Nadel, vorzugsweise im Abstand von der Nadelspitze ein Heizelement angeordnet sein. Das Heizelement ist so angeordnet, dass es Wärme an die wärmeleitende Außenoberfläche abgibt und so zu einer schnellen Erwärmung der gesamten Nadelaußenoberfläche bis zur Spitze führen.

Beispielsweise kann es sich bei dem Heizelement um einen Ring aus einem ferromagnetischen Material handeln, der induktiv erhitzt werden kann. In diesem Fall umfasst die Vorrichtung eine Spule zur Erzeugung eines magnetischen Wechselfelds. Ferner kann es sich bei dem Heizelement um ein elektrisches Widerstandselement handeln, das mit einem elektrischen Kontakt der Vorrichtung in Verbindung steht.

In einer Ausführungsform weist die Vorrichtung eine UV-Lichtquelle und/oder eine Düse zum Besprühen der Nadel mit Desinfektionsmittel auf. Die UV-Lichtquelle und die Düse können dazu dienen, die Nadel vor deren Einführung in die Befüllleitung oder -öffnung des Beutels zu sterilisieren.

In einer Ausführungsform weist die Vorrichtung eine Präparationskammer auf, welche die Nadel aufnehmen kann. Die Präparationskammer kann eine Abdeckung umfassen, um die In Parkposition befindliche Nadel in der Kammer einschließen zu können. Innerhalb der Präparationskammer können die UV-Lichtquelle oder die Düse zum Besprühen der Nadel mit Desinfektionsmittel angeordnet sein.

Es kann vorgesehen sein, dass die Vorrichtung ausgebildet ist, die Nadel in axialer Richtung zwischen einer Parkposition und einer Befüllposition zu bewegen. Die Linearbewegung zwischen Parkposition und Befüllposition soll in der Anwendung mit einem Einführen der Nadel in die Befüllleitung oder -öffnung und einem Ausziehen der Nadel aus der Befüllleltung oder -öffnung einhergehen. Die Parkposition kann innerhalb der gegebenenfalls vorhandenen Präparationskammer liegen.

In einer Ausführungsform ist vorgesehen, dass die Vorrichtung Klemmbacken umfasst, die ausgebildet sind, die Befüllleitung bzw. -öffnung des Beutels zusammenzudrücken. Anhand der Klemmbacken kann die Befüllleitung bzw. -öffnung versiegelt werden, wenn zuvor die Innenseiten der Befüllleitung bzw.- öffnung durch Erhitzen der Nadelaußenwand oberflächlich aufgeschmolzen wurden. Da ein Aufschmelzen durch die Klemmbacken selbst nicht erforderlich ist, können diese ohne Heizelemente ausgebildet werden.

In einer Ausführungsform ist vorgesehen, dass die Vorrichtung mehrere vorzugsweise baugleiche Befüllplätze mit Nadeln aufweist, wobei vorzugsweise vorgesehen ist, dass die Befüllplätze auf einer Karuselldrehstation oder fließbandartig nebeneinander angeordnet sind.

Die mehreren Befüllplätze und Nadeln sind vorzugsweise alle identisch ausgebildet. Die Befüllplätze können jeweils eine Präparationskammer umfassen. Durch eine Verwendung mehrerer Nadeln kann der Durchsatz erhöht werden. Im Falle einer Karuselldrehstation kann jeder Befüllplatz seine Rotationsstellung im Takt ändern, wobei jeder Rotationsstellung ein Schritt im Befüllzyklus zugeordnet ist, z.B., Fixierung des Beutels am Befüllplatz, Einführen der Nadel in die Befüllleitung oder - öffnung, Einleiten der Flüssigkeit, Erhitzen der Nadel und Anschmelzen der Innenflächen der Befüllleitung oder -öffnung, Ausziehen der Nadel und Klemmen der Befüllleitung oder -öffnung zur Versiegelung, Abkopplung des Beutels, Desinfektion der Nadel in der Parkposition, etc. Da die Nadeln bei geeigneter Taktwahl in dieser Variante immer in Verwendung sind und Beutel immer an derselben Stelle an die Befüllplätze herangeführt und von diesen abgezogen werden, kann eine derartige Anordnung aus produktionstechnischer Sicht besonders vorteilhaft sein.

Die gilt entsprechend für die Verwendung einer fließbandartigen Anordnung der Befüllplätze.

Vor dem eingangs genannten Hintergrund betrifft die Erfindung ferner ein Verfahren zum Befüllen eines Lösungsbeutels für die Dialyse mit einer Flüssigkeit anhand der erfindungsgemäßen Vorrichtung, gemäß Anspruch 8. Die Nadel wird in eine Befüllleitung oder - öffnung des Lösungsbeutels eingeführt, Flüssigkeit in den Lösungsbeutel abgibt und sodann wieder aus der Befüllleitung oder -öffnung herausgezogen .

Dabei ist die Temperatur an der Außenseite der Außenwand der Nadel größer als an der Innenseite der Innenwand.

Bei der durch den Flüssigkeitskanal der Nadel abgegebenen Flüssigkeit kann es sich je nach Konzept um eine vorgefertigte Dialyselösung, um eine Teillösung oder ein Lösungskonzentrat sowie um steriles und deionisiertes Wasser handeln.

Die Befüllleitung oder -öffnung des Beutels kann vor dem Einführen der Nadel mit beispielsweise einer Membran verschlossen sein und der Verschluss beim Einführen der Nadel in die Befüllleitung oder -öffnung durchstochen werden. Zum Einführen und Herausziehen der Nadel ist vorzugsweise vorgesehen, dass die Nadel in axialer Richtung zwischen einer Parkposition und einer Befüllposition bewegt wird. Auch eine Bewegung des Beutels bzw. der Befüllleitung oder -öffnung ist aber denkbar und von der Erfindung umfasst.

In einer Ausführungsform ist vorgesehen, dass der Befüllvorgang mit derselben Nadel vielfach wiederholt wird, um eine Vielzahl an Lösungsbeuteln zu befüllen. Die Nadel wird also als Mehrwegnadel verwendet. Die Nadel kann gleichwohl auswechselbar an der Vorrichtung befestigt sein, um nach einer Lebensdauer von beispielsweise einigen Tagen oder mehreren Hundert Beuteln einen Austausch vornehmen zu können.

In einer Ausführungsform ist vorgesehen, dass die Außenwand der Nadel vor dem Einführen der Nadel in die Befüllleitung oder -öffnung erhitzt wird, vorzugsweise auf einer Temperatur von mehr als 80°C. Das Erhitzen führt zu einer Desinfektion der Nadelaußenoberfläche und der angrenzenden Beutelbereiche. Bevorzugt ist ein Erhitzen auf eine Temperatur von größer 100°C, vorzugsweise über 121 °C.

Die Nadelaußenwand wird vorzugsweise nur kurz auf dieser Temperatur gehalten, beispielsweise weniger als 30 Sekunden und vorzugsweise weniger als 10 Sekunden. Vor dem Einführen der Nadel in die Befüllleitung oder -öffnung soll diese wieder abgekühlt sein, jedenfalls auf eine Temperatur von unter 100°C und vorzugsweise auf eine Temperatur von unter 80°C.

Alternativ oder zusätzlich kann die Außenwand der Nadel vor dem Einführen der Nadel in die Befüllleitung oder -öffnung mit UV-Licht bestrahlt und/oder mit einem Desinfektionsmittel besprüht werden.

Durch die vielfache Wiederholung des Befüllvorgangs entspricht ein Erhitzen oder anderweitiges Desinfizieren vor einem Befüllvorgang gleichzeitig einem Erhitzen bzw. anderweitigen Desinfizieren nach einem vorhergehenden Befüllvorgang. Das Erhitzen bzw. anderweitige Desinfizieren kann zwischen allen Befüllvorgängen oder immer nach Erledigung einer bestimmten Anzahl an Befüllvorgängen, beispielsweise nach Erledigung von zwei, fünf oder zehn Befüllvorgängen erfolgen.

In einer Ausführungsform kann die Nadel zwischen den Befüllvorgängen zum Desinfizieren in eine Präparationskammer eingebracht werden, entweder durch Einziehen der Nadel in die Parkposition oder durch Überstülpen der Kammer. Die Kammer kann gegebenenfalls geschlossen werden.

In einer Ausführungsform ist vorgesehen, dass die Außenwand der Nadel erhitzt wird, während die Nadel in der Befüllleitung oder -öffnung eingeführt ist, um die Innenoberflächen der Befüllleitung oder -öffnung durch die Hitzeentwicklung der Nadel aufzuschmelzen, und dass nach dem Herausziehen der Nadel die Befüllleitung oder -öffnung zusammengedrückt wird, um die Befüllleitung oder -öffnung durch Verbindung von deren aufgeschmolzenen Innenoberflächen zu versiegeln.

Vorzugsweise wird die Nadel dabei auf eine Temperatur erhitzt, die zu einem oberflächlichen Aufweichen bzw. Schmelzen der Innenoberfläche der Befüllleitung oder -öffnung führt. Bevorzugt ist ein Erhitzen auf eine Temperatur von größer 100°C, größer 120°C oder größer 150°C.

Die Befüllleitung oder -öffnung ist typischerweise aus einem thermoplastischen Kunststoff gefertigt. Es kann vorgesehen sein, dass der thermoplastische Kunststoff der Befüllleitung oder -öffnung direkt aufgeschmolzen wird oder es kann zum Zwecke der Verschweißung an der Innenoberfläche der Befüllleitung oder -öffnung ein thermoplastischer Kunststoff aufgetragen sein, der eine niedrigere Schmelztemperatur aufweist. Auch während des Erhitzens kann die Befüllleitung oder -öffnung bereits zusammengedrückt und mithin die Innenoberfläche des Befüllschlauchs oder -öffnung an die erhitzte Nadel gedrückt werden, um das Schmelzen zu beschleunigen.

In einer Ausführungsform Ist vorgesehen, dass die Nadelaußenwand induktiv oder durch Durchfluss von elektrischem Strom erhitzt wird. Das Erhitzen kann dabei unmittelbar, also durch induktives Erhitzen der Nadelaußenwand selbst bzw. durch Durchfluss von elektrischem Strom durch die Nadelaußenwand selbst erfolgen. Alternativ kann vorgesehen sein, dass ein mit der Nadelaußenwand in thermischem Kontakt stehendes Heizelement induktiv oder durch Durchfluss von elektrischem Strom erhitzt wird. Beispiele für das Heizelement umfassen einen metallischen Ring, der im Abstand von der Nadelspitze am Nadelschaft angeordnet ist.

In einer Ausführungsform ist vorgesehen, dass mehrere vorzugsweise baugleiche Befüllplätze der Vorrichtung gleichzeitig verwendet werden, wobei dieselben Verfahrensschritte an den unterschiedlichen Befüllplätzen zeitversetzt oder gleichzeitig durchgeführt werden.

Beispielsweise kann vorgesehen sein, dass an einem ersten Befüllplatz die erste Nadel in die erste Befüllleitung oder -öffnung eines ersten Beutels eingeführt wird und der erste Beutel befüllt wird, während an einem zweiten Befüllplatz die in einer zweiten Befüllleitung oder -öffnung befindliche zweite Nadel erhitzt wird, um die Innenoberflächen der zweiten Befüllleitung oder -öffnung aufzuschmelzen. An einem weiteren, dritten Befüllplatz kann zur gleichen Zeit eine dritte Nadel aus einer dritten Befüllleitung oder -öffnung eines dritten Beutels gezogen werden und die dritte Befüllleitung oder -öffnung zusammengedrückt werden, um die dritte Befüllleitung oder -öffnung durch Verbindung zuvor aufgeschmolzener Innenoberflächen zu versiegeln. An einem vierten Befüllplatz kann gleichzeitig die vierte Nadel in ihrer Parkposition desinfiziert werden und an einem fünften Befüllplatz eine fünfte Nadel abkühlen.

Die Befüllplätze können an einer rotierbaren Karuselldrehstation angeordnet sein, die zwischen zwei Verfahrensschritten jeweils um einen Winkel gedreht wird, der einem der Anzahl der Befüllplätzen entsprechenden Bruchteil einer ganzen Drehung entspricht. Auch eine Anordnung mehrerer Befüllplätze nebeneinander ist denkbar und von der Erfindung umfasst.

Von besonderer Relevanz ist die Erfindung im Zusammenhang mit dezentralen und gegebenenfalls mobilen Einheiten zur Herstellung von Dialyselösungen vor Ort und zur direkten Abgabe an den Patienten. In derartigen Einheiten ist es typischerweise schwieriger, die Sterilität bei der Befüllung der Lösungsbeutel zu gewährleisten.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Weitere Einzelheiten und Vorteile der Erfindung ergeben sich aus den nachfolgend anhand der Figuren beschriebenen Ausführungsbeispiele. In den Figuren zeigen:
- Figur 1:: eine schematische Darstellung einer doppelwandigen Nadel einer erfindungsgemäßen Vorrichtung;
- Figur 2:: eine schematische Darstellung mehrerer Verfahrensschritte eines erfindungsgemäßen Verfahrens; und
- Figur 3:: eine schematische Darstellung mehrerer Verfahrensschritte einer alternativen Variante eines erfindungsgemäßen Verfahrens.

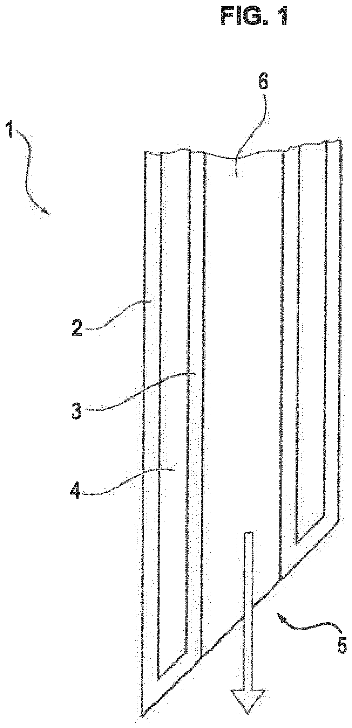

In Figur 1 ist eine doppelwandige Nadel 1 zur Verwendung In einer erfindungsgemäßen Vorrichtung und einem erfindungsgemäßen Verfahren gezeigt. Es handelt sich um eine doppelwandige Nadel 1 aus rostfreiem Edelstahl. Zwischen der Außenwand 2 und der Innenwand 4 der Nadel 1 ist ein Hohlraum vorgesehen, der vakuumisoliert ist. Die Spitze 5 der Nadel 1 ist angeschrägt, um Membranen oder andere Verschlüsse in der Befüllleitung oder -öffnung von Lösungsbeuteln besser durchstoßen zu können.

Bei der Nadel 1 handelt es sich um eine Mehrwegnadel, die bestimmungsgemäß für die Befüllung von einer Vielzahl an Lösungsbeuteln eingesetzt wird. Sie ist in einer nicht näher dargestellten Weise austauschbar am Befüllplatz einer Vorrichtung zum Befüllen von Lösungsbeuteln für die Dialyse angeordnet. Der Flüssigkeitskanal 6 dient zur Einleitung von Flüssigkeit in eine Befüllleitung oder -Öffnung des Beutels.

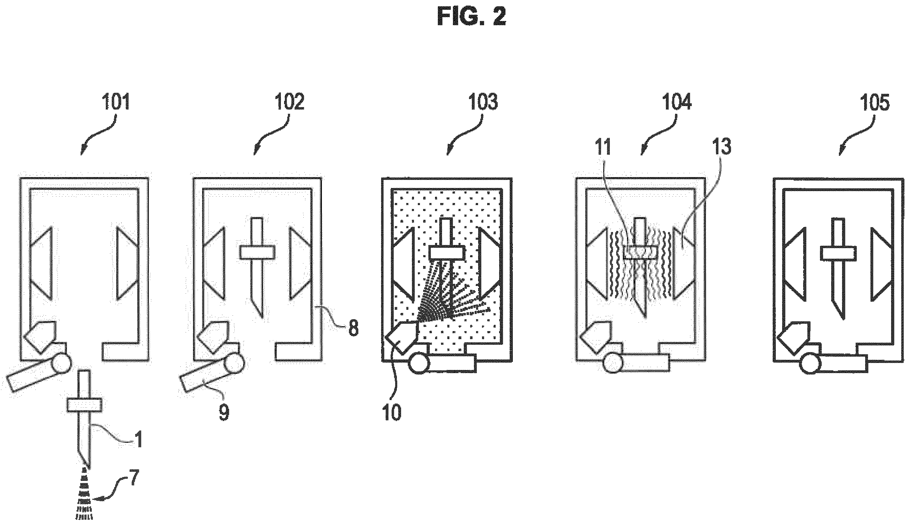

In Figur 2 ist eine schematische Darstellung mehrerer Verfahrensschritte einer Variante eines erfindungsgemäßen Verfahrens dargestellt. Die dort gezeigte Nadel 1 ist so ausgebildet, wie dies im Zusammenhang mit Figur 1 beschrieben wurde.

In einem ersten Verfahrensschritt, dem Befüllschritt 101, wird die Nadel in eine ausgefahrene Befüllposition überführt, wobei sie in einen nicht näher dargestellten Befüllschlauch eines nicht näher dargestellten Lösungsbeutels eindringt und eine Membran durchstößt. In der Figur ist die Nadel 1 in ihrer Befüllposition gezeigt. Aus dem Flüssigkeitskanal der Nadel fließt Flüssigkeit 7 wie beispielsweise eine fertig gemischte Dialyselösung durch den Flüssigkeitskanal der Nadel 1 in eine Befüllleitung oder -öffnung des Beutels.

In einem folgenden Verfahrensschritt, dem Ausziehschritt 102, wird die Nadel 1 aus der Befüllleitung oder -öffnung des Beutels herausgezogen und durch axiale translatorische Bewegung in eine Parkposition übergeführt, in welcher die Nadel 1 vollständig in einer Präparationskammer 8 aufgenommen Ist. Die Präparationskammer umfasst eine Luke 9 zum Verschließen der Kammer 8 an der Stelle, an der die Nadel 1 aus der Befüllposition eingezogen wurde.

In einem weiteren Verfahrensschritt, dem Spraydesinfektionsschritt 103, wird bei geschlossener Luke 9 die Nadel 1 innerhalb der Kammer 8 mit Desinfektionsmittel besprüht. Zu diesem Zweck Ist in der Kammer 8 eine Düse 10 angeordnet.

Sodann wird In einem Hitze- und UV-Desinfektionsschritt 104 bei geschlossener Luke 9 die Nadel 1 innerhalb der Kammer 8 mit UV-Licht bestrahlt. Zu diesem Zweck sind in der Kammer UV-Lichtquellen 13 angeordnet. Gleichzeitig wird eine nicht näher dargestellte, neben der Kammer 8 angeordnete Spule mit hochfrequentem Wechselstrom beaufschlagt, um einen Edelstahlring 11 induktiv zu erhitzen, der am Schaft der Nadel 1 angeordnet ist und einstückig mit der Außenwand 2 in Verbindung steht. Ausgehend von diesem Ring 11 erhitzt sich die Außenwand 2 der gesamten Nadel auf eine Solltemperatur von 120°C. Sobald die Solltemperatur erreicht wurde, wird das induktive Erhitzen ausgesetzt.

In einem letzten Schritt, dem Abkühlschritt 105 ruht die Nadel 1 innerhalb der Kammer 8, sodass sich die Außenwand 2 auf Raumtemperatur oder auf knapp über Raumtemperatur abkühlen kann. Im Anschluss beginnt das Verfahren von vorne.

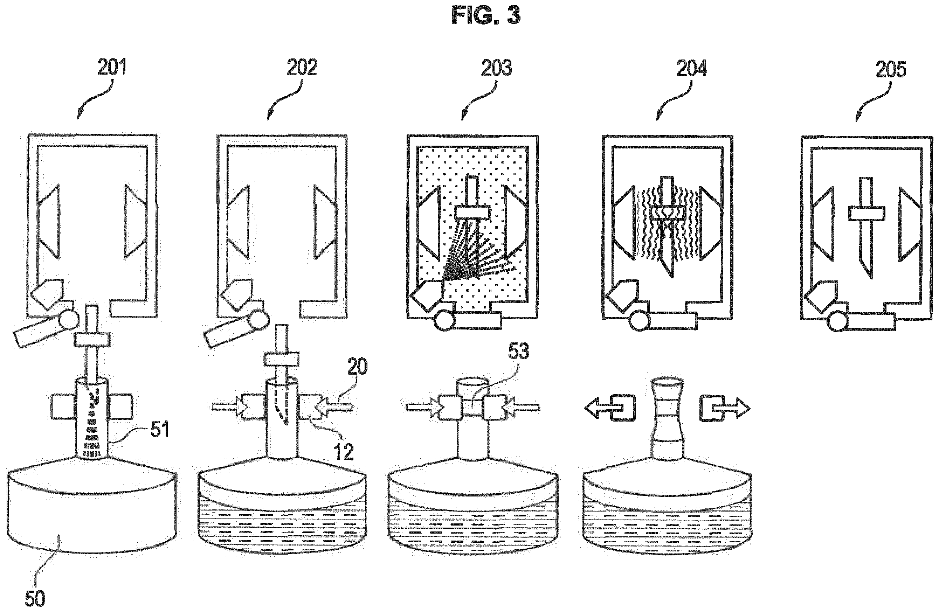

In Figur 3 ist eine schematische Darstellung mehrerer Verfahrensschritte einer anderen Variante eines erfindungsgemäßen Verfahrens dargestellt. Die dort gezeigte Nadel 1 ist wiederum so ausgebildet, wie dies im Zusammenhang mit Figur 1 beschrieben wurde.

Der Befüllschritt 201 dieses Verfahrens entspricht dem Befüllschritt 101 des in Figur 2 dargestellten Verfahrens.

Auf diesen Schritt folgt im Verfahren der Figur 3 aber ein Schmelzschritt 202, in welchem die Nadel 1 sich weiterhin in ihrer Befüllposition befindet und in dem, diesmal dargestellte, Befüllschlauch 51 des Lösungsbeutels 50 aufgenommen ist. In dieser Position wird die Außenwand 2 der Nadel durch induktives Erhitzen des Ringes 11 erwärmt, wie oben im Zusammenhang mit Schritt 104 der Figur 2 beschrieben. Die nicht dargestellte bestromte Spule befindet sich hier außerhalb der Kammer 8. Bereits während des Erhitzens wird ein Paar an Klemmbacken 12 der Vorrichtung von außen gegen den Befüllschlauch 51 gedrückt, sodass ein aufschmelzen der Innenoberfläche des Befüllschlauchs 51 an der erhitzten Außenwand 2 der Nadel begünstigt wird. Die Klemmbewegung der Klemmbacken 12 ist in der Figur anhand der Pfeile 20 symbolisiert.

Auf den Schmelzschritt 202 folgt ein Versiegelungsschritt 203, in dem die Nadel 1 aus der Befüllleitung oder -öffnung des Beutels herausgezogen und, wie in Schritt 102 der Figur 2, durch axiale translatorische Bewegung in eine Parkposition übergeführt wird, in welcher die Nadel 1 vollständig in einer Präparationskammer 8 aufgenommen ist. Dort erfolgt eine Spraydesinfektion der Nadel, wie In Schritt 103 der Figur 2. Gleichzeitig wird das Paar an Klemmbacken 12 nun vollständig zusammengeklemmt, um die zuvor aufgeschmolzenen Bereiche an der Innenoberfläche des Befüllschlauchs 51 zu einem Schmelzsiegel 52 zu verbinden und den Befüllschlauch 51 so zu versiegeln.

Es folgen ein Hitze- und UV-Desinfektionsschritt 204 und ein Abkühlschritt 205, welche wiederum den entsprechenden Schritten 104 und 105 des Verfahrens gemäß Figur 2 entsprechen.

Es ist vorgesehen, dass alle Schritte 101 bis 105 bzw. 201 bis 205 jeweils eine identische Zeitdauer in Anspruch nehmen. Die erfindungsgemäße Vorrichtung weist fünf baugleiche Befüllplätze mit Nadel 1 und Kammer 8 auf, die gleichmäßig über den Umfang eines Drehkarusells verteilt angeordnet sind. Das Drehkarusell dreht sich nach Ablauf der Zeitspanne, die der Dauer eines Verfahrensschritts entspricht, immer um 72°, sodass die einzelnen Befüllplätze nach Vollendung eines Verfahrensschritts immer die Position eines benachbarten Befüllplatzes einnehmen, bevor der nachfolgende Verfahrensschritt eingeleitet wird. So werden dieselben Verfahrensschritte immer an derselben Position in der Vorrichtung durchgeführt, wobei sich die unterschiedlichen Befüllplätze, ggf. mit unterschiedlichen angeschlossenen Beuteln, bei Durchführung eines bestimmten Verfahrensschritts immer an derselben, bestimmten Position der Vorrichtung befinden.

In einer Variante kann nur die Nadel 1 durch Drehung des Karussells ihre Position wechseln und die Kammer 8 kann stationär angeordnet sein, sodass einzelne Elemente wie beispielsweise die UV-Lichtquelle und die Spule nicht redundant ausgeführt sein müssen.

## Patentansprüche

1. Vorrichtung zum Befüllen eines Lösungsbeutels für die Dialyse mit einer medizinischen Flüssigkeit, wobei die Vorrichtung eine Nadel (1) zum Einführen in eine Befüllleitung oder -öffnung des Beutels umfasst,
**dadurch gekennzeichnet,**
**dass** es sich bei der Nadel um eine doppelwandige Nadel mit einer vorzugsweise wärmeleitenden Außenwand (2) und mit einer Innenwand (4) handelt, wobei zwischen der Außenwand und der Innenwand eine thermische Isolierung vorhanden ist, wobei ein Heizelement vorgesehen ist, das ausgebildet ist, die Außenseite der Außenwand auf eine Temperatur oberhalb der Temperatur der Innenseite der Innenwand zu erhitzen.

2. Vorrichtung nach Anspruch 1, **dadurch gekennzeichnet, dass** die Außenwand der Nadel aus Metall besteht und/oder dass es sich bei der thermischen Isolierung um eine Vakuumisolierung oder um ein Luft- oder Gaspolster handelt und/oder dass an der Außenwand der Nadel vorzugweise im Abstand von der Nadelspitze ein Heizelement angeordnet ist.

3. Vorrichtung nach Anspruch 1 oder 2, **dadurch gekennzeichnet, dass** es sich bei der Heizeinrichtung um eine elektrische Heizeinrichtung handelt.

4. Vorrichtung nach einem der vorhergehenden Ansprüche, **dadurch gekennzeichnet, dass** die Vorrichtung Klemmbacken (12) umfasst, die ausgebildet sind, die Befüllleitung oder -öffnung des Beutels nach dessen Befüllung zusammenzudrücken.

5. Vorrichtung nach einem der vorhergehenden Ansprüche, **dadurch gekennzeichnet, dass** die Vorrichtung einen Befüllplatz aufweist, der die Nadel umfasst.

6. Vorrichtung nach einem der vorhergehenden Ansprüche, **dadurch gekennzeichnet, dass** die Vorrichtung mehrere vorzugsweise baugleiche Befüllplätze mit Nadeln aufweist, wobei vorzugsweise vorgesehen ist, dass die Befüllplätze auf einer Karuselldrehstation angeordnet sind.

7. Vorrichtung nach einem der vorhergehenden Ansprüche, **dadurch gekennzeichnet, dass** die Befüllleitung oder -öffnung aus einem Material besteht, das durch Wärmeeinwirkung flüssigkeitsdicht versiegelbar ist.

8. Verfahren zum Befüllen eines Lösungsbeutels für die Dialyse mit einer Flüssigkeit anhand einer Vorrichtung nach einem der vorhergehenden Ansprüche,
**dadurch gekennzeichnet,**
**dass** die Nadel in eine Befüllleitung oder -öffnung des Lösungsbeutels eingeführt wird, Flüssigkeit in den Lösungsbeutel abgibt und sodann wieder aus der Befüllleitung oder -öffnung herausgezogen wird, wobei die Temperatur der Außenseite der Außenwand der Nadel über der Temperatur der Innenseite der Innenwand der Nadel liegt, die mit der Flüssigkeit in Kontakt steht, die in den Lösungsbeutel einströmt.

9. Verfahren nach Anspruch 8, **dadurch gekennzeichnet, dass** der Vorgang des Befüllens mit derselben Nadel mehrfach wiederholt wird, um eine Mehrzahl an Lösungsbeuteln zu befüllen.

10. Verfahren nach Anspruch 8 oder 9, **dadurch gekennzeichnet, dass** die Außenwand der Nadel vor und/oder während des Einführens der Nadel in die Befüllleitung oder -öffnung erhitzt wird, vorzugsweise auf eine Temperatur von mehr als 80°C.

11. Verfahren nach einem der Ansprüche 8 bis 10, **dadurch gekennzeichnet, dass** die Außenwand der Nadel erhitzt wird, bevor oder während die Nadel in der Befüllleitung oder -öffnung eingeführt ist, so dass die Innenoberfläche der Befüllleitung oder -öffnung durch die Hitzeentwicklung der Nadel aufgeschmolzen wird, und dass nach dem Herausziehen der Nadel die Befüllleitung oder -öffnung zusammengedrückt wird, um die Befüllleitung oder - öffnung durch Verbindung von deren aufgeschmolzenen Innenoberflächen flüssigkeitsdicht zu versiegeln.

12. Verfahren nach Anspruch 10 oder 11, **dadurch gekennzeichnet, dass** die Nadelaußenwand induktiv oder durch Durchfluss von elektrischem Strom erhitzt wird.

13. Verfahren nach einem der Ansprüche 8 bis 12, **dadurch gekennzeichnet, dass** mehrere vorzugsweise baugleiche Befüllplätze der Vorrichtung gleichzeitig verwendet werden, wobei dieselben Verfahrensschritte an den unterschiedlichen Befüllplätzen zeitversetzt oder zeitgleich duchgeführt werden.

## Claims

1. An apparatus for filling a solution bag for dialysis with a medical liquid, wherein the apparatus comprises a needle (1) for introduction into a filling line or filling opening of the bag,
**characterized in that**
the needle is a double wall needle having a preferably thermally conductive outer wall (2) and having an inner wall (4), with a thermal insulation being present between the outer wall and the inner wall; and with a heating element being provided that is configured to heat the outer side of the outer wall to a temperature above the temperature of the inner side of the inner wall.

2. An apparatus in accordance with claim 1, **characterized in that** the outer wall of the needle consists of metal; and/or **in that** the thermal insulation is a vacuum insulation or an air cushion or gas cushion; and/or **in that** a heating element is arranged at the outer wall of the needle, preferably at a distance from the needle tip.

3. An apparatus in accordance with claim 1 or claim 2, **characterized in that** the heating device is an electric heating device.

4. An apparatus in accordance with one of the preceding claims, **characterized in that** the apparatus comprises clamping jaws (12) that are configured to compress the filling line or filling opening of the bag after its filling.

5. An apparatus in accordance with one of the preceding claims, **characterized in that** the apparatus has a filling station that comprises the needle.

6. An apparatus in accordance with one of the preceding claims, **characterized in that** the apparatus has a plurality of filling stations of preferably the same construction and having needles, with provision preferably being made that the filling stations are arranged on a carousel rotation station.

7. An apparatus in accordance with one of the preceding claims, **characterized in that** the filling line or filling opening consists of a material that can be sealed in a liquid-tight manner by the effect of heat.

8. A method for filling a solution bag for dialysis with a liquid using an apparatus in accordance with one of the preceding claims,
**characterized in that**
the needle is introduced into a filling line or filling opening of the solution bag, outputs liquid into the solution bag, and is then withdrawn from the filling line or filling opening again, with the temperature of the outer side of the outer wall of the needle being above the temperature of the inner side of the inner wall of the needle that is in contact with the liquid that flows into the solution bag.

9. A method in accordance with claim 8, **characterized in that** the process of filling is repeated multiple times using the same needle to fill a plurality of solution bags.

10. A method in accordance with claim 8 or claim 9, **characterized in that** the outer wall of the needle is heated before and/or during the introduction of the needle into the filling line or filling opening, preferably to a temperature of more than 80°C.

11. A method in accordance with any one of the claims 8 to 10, **characterized in that** the outer wall of the needle is heated before or while the needle is introduced in the filling line or filling opening such that the inner surface of the filling line or filling opening is melted by the heat development of the needle; and **in that** the filling line or filling opening is compressed after the withdrawal of the needle to seal the filling line or filling opening in a liquid-tight manner by connecting its melted inner surfaces.

12. A method in accordance with claim 10 or claim 11, **characterized in that** the outer needle wall is heated inductively or by throughflow of electric current.

13. A method in accordance with one of the claims 8 to 12, **characterized in that** a plurality of filling stations of the apparatus of preferably the same construction are used simultaneously, with the same method steps being carried out with a time offset or at the same time at the different filling stations.

## Revendications

1. Dispositif de remplissage d'une poche de solution pour dialyse avec un liquide médical, le dispositif comprenant une aiguille (1) destinée à l'introduction dans un conduit ou une ouverture de remplissage de la poche,
**caractérisé en ce que**
l'aiguille est une aiguille à double paroi avec une paroi extérieure (2) de préférence thermoconductrice et avec une paroi intérieure (4), une isolation thermique se trouvant entre la paroi extérieure et la paroi intérieure, un élément chauffant étant prévu, lequel est conçu pour chauffer le côté extérieur de la paroi extérieure à une température supérieure à la température du côté intérieur de la paroi intérieure.

2. Dispositif selon la revendication 1, **caractérisé en ce que** la paroi extérieure de l'aiguille est constituée de métal et/ou **en ce que** l'isolation thermique est une isolation par le vide ou un matelas d'air ou de gaz et/ou **en ce qu'**un élément chauffant est disposé sur la paroi extérieure de l'aiguille, de préférence à distance de la pointe de l'aiguille.

3. Dispositif selon la revendication 1 ou 2, **caractérisé en ce que** le dispositif chauffant est un dispositif chauffant électrique.

4. Dispositif selon l'une des revendications précédentes, **caractérisé en ce que** le dispositif comprend des mâchoires de serrage (12), qui sont conçues pour comprimer le conduit ou l'ouverture de remplissage de la poche après le remplissage de cette dernière.

5. Dispositif selon l'une des revendications précédentes, **caractérisé en ce que** le dispositif comporte un emplacement de remplissage, qui comprend l'aiguille.

6. Dispositif selon l'une des revendications précédentes, **caractérisé en ce que** le dispositif comporte plusieurs emplacements de remplissage, de préférence de construction identique, avec des aiguilles, les emplacements de remplissage étant de préférence prévus disposés sur un poste rotatif de type carrousel.

7. Dispositif selon l'une des revendications précédentes, **caractérisé en ce que** le conduit ou l'ouverture de remplissage est constitué(e) d'un matériau qui peut être scellé de manière étanche aux liquides par action de la chaleur.

8. Procédé de remplissage d'une poche de solution pour dialyse avec un liquide à l'aide d'un dispositif selon l'une des revendications précédentes,
**caractérisé en ce que**
l'aiguille est introduite dans un conduit ou une ouverture de remplissage de la poche de solution, délivre du liquide dans la poche de solution puis est retirée du conduit ou de l'ouverture de remplissage, la température du côté extérieur de la paroi extérieure de l'aiguille étant supérieure à la température du côté intérieur de la paroi intérieure de l'aiguille, qui est en contact avec le liquide qui pénètre dans la poche de solution.

9. Procédé selon la revendication 8, **caractérisé en ce que** le processus du remplissage est répété plusieurs fois avec la même aiguille pour remplir une pluralité de poches de solution.

10. Procédé selon la revendication 8 ou 9, **caractérisé en ce que** la paroi extérieure de l'aiguille est chauffée avant et/ou pendant l'introduction de l'aiguille dans le conduit ou l'ouverture de remplissage, de préférence à une température supérieure à 80 °C.

11. Procédé selon l'une des revendications 8 à 10, **caractérisé en ce que** la paroi extérieure de l'aiguille est chauffée avant ou pendant l'introduction de l'aiguille dans le conduit ou l'ouverture de remplissage, de telle sorte que la surface intérieure du conduit ou de l'ouverture de remplissage est fondue par le dégagement de chaleur de l'aiguille et **en ce que**, après le retrait de l'aiguille, le conduit ou l'ouverture de remplissage est comprimé(e) pour sceller le conduit ou l'ouverture de remplissage de manière étanche aux liquides par la liaison de ses surfaces intérieures fondues.

12. Procédé selon la revendication 10 ou 11, **caractérisé en ce que** la paroi extérieure de l'aiguille est chauffée par induction ou par passage d'un courant électrique.

13. Procédé selon l'une des revendications 8 à 12, **caractérisé en ce que** plusieurs emplacements de remplissage, de préférence de construction identique, du dispositif sont utilisés simultanément, les mêmes étapes de procédé étant exécutées avec un décalage dans le temps ou en même temps aux différents emplacements de remplissage.
